# EUROPEAN PATENT APPLICATION

(11) **EP 4 571 401 A1**
(43) Date of publication of application: **18.06.2025**
(21) Application number: 23852349.2
(22) Date of filing: 24.07.2023
(51) Int. Cl.: G02C 13/00, A61B 3/028, A61B 3/036, G02C 7/02, G02C 7/06

(54) **EYEGLASS LENS DETERMINATION METHOD, AND EYEGLASS LENS DETERMINATION ASSISTANCE SYSTEM**

(30) Priority: 12.08.2022 JP 2022128803
(71) Applicant: Hoya Lens Thailand Ltd., Pathumthani 12130 (TH)
(72) Inventor: MATSUOKA Shohei, Tokyo 160-8347 (JP)
(74) Representative: Betten & Resch
(86) International application number: PCT/JP2023/026965
(87) International publication number: WO 2024/034373

(57) **Abstract**

There is provided a method for determining a spectacle lens, the method including: preparing multiple blurred images in which an amount of added aberration is varied with respect to a predetermined original image; simultaneously presenting the multiple blurred images to a subject to compare appearances of them, then obtaining a subjective response of the subject, to thereby measure a subject's sensitivity to aberration; and determining a spectacle lens suitable for the subject based on the subject's sensitivity to aberration.

## Description

### Background

### Technical Field

The present invention relates to a method for determining a spectacle lens and a system for supporting determination of a spectacle lens.

### Description of related art

Various design methods have been proposed to realize a spectacle lens that is suited to the characteristics of an individual wearer. For example, according to Patent document 1, there is provided a method for designing a spectacle lens, the method including: presenting multiple blurred images created by applying different degrees of blurring (blurring level) to an original image, allowing a wearer to view them, acquiring information regarding a wearer's sensitivity to blur, and designing a spectacle lens based on the information regarding the wearer's sensitivity to blur.

Further, for example, according to Patent document 1, there is provided a method for designing a spectacle lens, the method including: displaying an image on a display device while maintaining a positional relationship between a subject's face and the display device, obtaining information evaluating a subject's visual sensitivity based on a subject's impression of viewing the image, and designing a spectacle lens based on the information obtained from evaluating the sensitivity.

### Prior art document

### Patent document

[Patent Document 1] International Publication No. 2018/101015
[Patent Document 2] International Publication No. 2019/009034

### Summary of the invention

### Problem to be solved by the invention

An object of one embodiment of the present invention is to provide a technique for determining a spectacle lens suitable for a subject, in consideration of a subject's sensitivity to aberration.

### Means for solving the problem

According to a first aspect of the present invention, there is provided a method for determining a spectacle lens, the method including:
preparing multiple blurred images in which an amount of added aberration is varied with respect to a predetermined original image;
simultaneously presenting the multiple blurred images to a subject to compare appearances of them, then obtaining a subjective response of the subject, to thereby measure a subject's sensitivity to aberration; and
determining a spectacle lens suitable for the subject based on the subject's sensitivity to aberration.

According to a second aspect of the present invention, there is provided the method for determining a spectacle lens of the first aspect,
wherein in the preparation of the multiple blurred images, multiple similar blurred images similar to the multiple blurred images are further prepared;
in the measurement of the subject's sensitivity to aberration, the subject is simultaneously presented with the multiple similar blurred images to compare their appearances, and a subjective response of the subject is obtained multiple times to thereby measure a consistency of the subjective response.

According to a third aspect of the present invention, there is provided the method for determining a spectacle lens of the first aspect,
wherein in the preparation of the multiple blurred images, difficulty level-varied multiple blurred images are further prepared so that a difference in appearance is easier or harder to distinguish than the above multiple blurred images; and
in the measurement of the subject's sensitivity to aberration, the subject is simultaneously presented with the difficulty level-varied multiple blurred images to compare appearances of them, and a subjective response of the subject is obtained multiple times, to thereby measure the subject's sensitivity to aberration.

According to a fourth aspect of the present invention, there is provided the method for determining a spectacle lens of the first aspect,
wherein in the preparation of the multiple blurred images, the original image is selected according to a characteristic direction of a decrease in spatial frequency characteristics due to the added aberration.

According to a fifth aspect of the present invention, there is provided the method for determining a spectacle lens of the first aspect,
wherein in the determination of the spectacle lens, a progressive power lens is determined.

According to a sixth aspect of the present invention, there is provided the method for determining a spectacle lens of the first aspect,
wherein in the measurement of the subject's sensitivity to aberration, the multiple blurred images are presented with a size such that a spatial frequency of main parts of the multiple blurred images is 3 CPD or more and 9 CPD or less.

According to a seventh aspect of the present invention, there is provided the method for determining a spectacle lens of the first aspect, wherein in the measurement of the subject's sensitivity to aberration, a subject's head is not fixed, and the multiple blurred images are presented at a distance of 0.3 m or more and 2 m or less from subject's eyes.

According to an eighth aspect of the present invention, there is provided a system for supporting determination of a spectacle lens, the system including:
a storage unit that stores multiple blurred images in which amounts of added aberrations are varied, with respect to a predetermined original image;
a display unit that simultaneously presents the multiple blurred images to a subject to compare appearances of them;
an input unit into which a subjective response of the subject is inputted;
a determination unit that determines a subject's sensitivity to aberration from the subjective response; and
an output unit from which information for determining a spectacle lens suitable for the subject is outputted based on the sensitivity to aberration.

### Advantage of the invention

According to one embodiment of the present invention, a spectacle lens suitable for a subject can be determined, in consideration of a subject's sensitivity to aberration.

### Brief description of the drawings

FIG. 1 is a flowchart showing an example of a method for determining a spectacle lens according to a first embodiment of the present invention.
FIG. 2 is a view illustrating an example of an original image and multiple blurred images according to the first embodiment of the present invention.
FIG. 3 is a view illustrating an example of a spot image created by an aberration added to the blurred images according to the first embodiment of the present invention.
FIG. 4 is a view illustrating an example of similar blurred images according to the first embodiment of the present invention.
FIG. 5 is a view for explaining characteristic directions of spatial frequency characteristics of an original image according to the first embodiment of the present invention.
FIG. 6A is an image with downward aberration added to an image (original image) of the text to be read rightward on the page.
FIG. 6B is an image with upward aberration added to an image (original image) of the text to be read rightward on the page.
FIG. 6C is an image with rightward aberration added to an image (original image) of the text to be read rightward on the page.
FIG. 6D is an image with leftward aberration added to an image (original image) of the text to be read rightward on the page.
FIG. 7A is an image with downward aberration added to an image (original image) of the text to be read downward on the page.
FIG. 7B is an image with upward aberration added to an image (original image) of the text to be read downward on the page.
FIG. 7C is an image with rightward aberration added to an image (original image) of the text to be read downward on the page.
FIG. 7D is an image with leftward aberration added to an image (original image) of the text to be read downward on the page.
FIG. 8A is a view illustrating a power distribution and an astigmatism distribution of a spectacle lens according to an example of the present invention.
FIG. 8B is a view illustrating a power distribution and an astigmatism distribution of the spectacle lens according to the example of the present invention.

### Detailed description of the invention

### <Finding obtained by the inventor>

First, the finding obtained by the inventor will be described. As described in Patent document 1, it is found that when multiple blurred images are presented in sequence, an evaluation may vary depending on a subject's memory, level of fatigue, adaptability, etc., and sensitivity to blur (hereinafter, this will also be referred to as sensitivity to aberration) may not be measured accurately. There is also a problem that it takes a long time to measure.

The present inventor has conducted extensive research into the above-described problems. As a result, it is found that by simultaneously presenting multiple blurred images to a subject to compare the appearances of them, the sensitivity to blur can be measured without being affected by a subjects' memory, fatigue, adaptability, etc. It is also found that the measurement time can be shortened. Further, since multiple blurred images are compared, a relative evaluation can be made, which has an advantage of making it easier for the subject to evaluate. Accordingly, it becomes easier to determine a spectacle lens that is suitable for the subject, in consideration of the subject's sensitivity to blur.

### [Details of the embodiment of the present invention]

Next, an embodiment of the present invention will be described below with reference to the drawings. The present invention is not limited to these examples, but is defined by the claims, and is intended to include all modifications within the meaning and scope of the claims.

### <First embodiment of the present invention>

### (1) Method for determining a spectacle lens

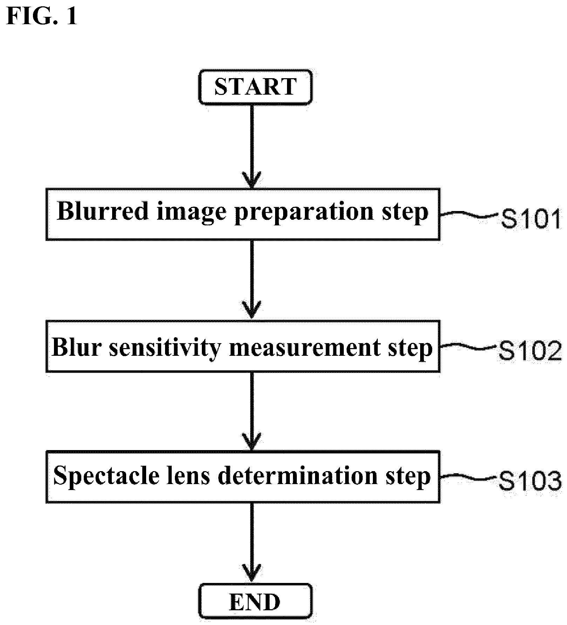

First, a method for determining a spectacle lens according to this embodiment will be described. FIG. 1 is a flowchart showing an example of the method for determining a spectacle lens according to this embodiment. As shown in FIG. 1, the method for determining a spectacle lens according to this embodiment includes, for example, a blurred image preparation step S101, a blur sensitivity measurement step S102, and a spectacle lens determination step S103. This embodiment shows a case of determining a progressive power lens suitable for a subject.

### (Blurred image preparation step S101)

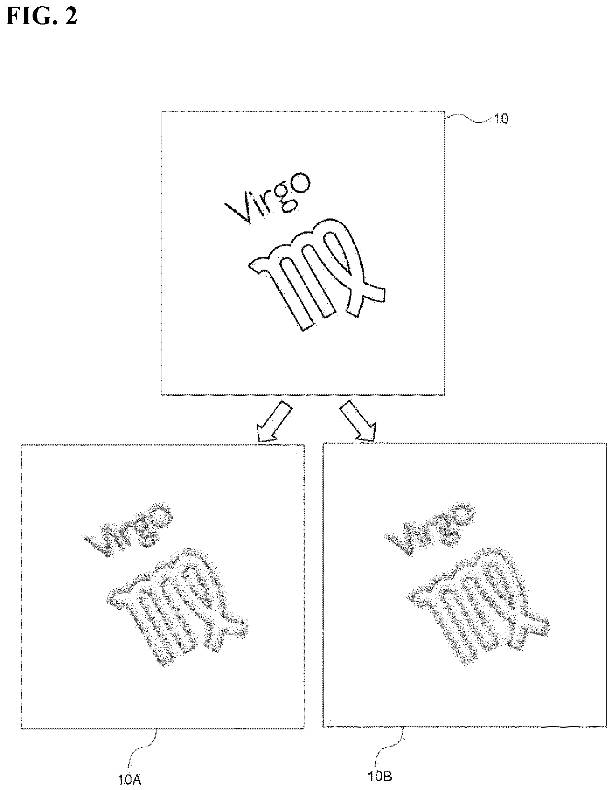

The blurred image preparation step S101 is, for example, a step of preparing multiple blurred images (in this embodiment, two blurred images) in which the amount of added aberration (preferably the amount only) is varied with respect to a predetermined original image. In this embodiment, the added aberration includes astigmatism, coma, trefoil aberration, etc. FIG. 2 illustrates an example of an original image and multiple blurred images. FIG. 2 illustrates a blurred image 10A in which predetermined amount of aberration is added, and a blurred image 10B in which larger amount of aberration than the blurred image 10A is added, with respect to an original image 10.

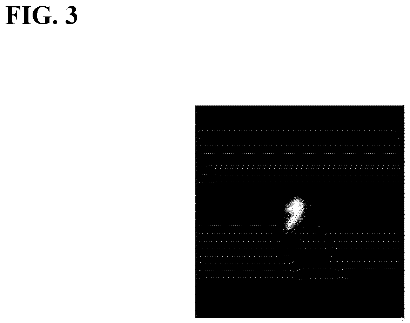

FIG. 3 illustrates an example of a spot image created by the aberration added to the blurred image. Also, FIG. 3 illustrates a spot image in an appearance of a trailing tail at the bottom left of the page. The blurred image added with aberration that creates such a spot image appears blurred in a lower left direction. In other words, the added aberration results in a noticeable (characteristic) decrease in spatial frequency characteristics in the lower left direction. Such a characteristic direction of the decrease in the spatial frequency characteristics due to the added aberration is referred to as a direction of aberration in this specification.

In the blurred image preparation step S101, it is preferable that the directions of the aberrations added to the multiple blurred images (for example, the blurred image 10A and the blurred image 10B) are substantially the same. This makes it easier for the subject to compare the appearances of the multiple blurred images. In this specification, the term "the directions of aberrations are substantially the same" includes a case where the directions are completely the same, as well as a case where there is a slight difference of ±15 degrees or less in the direction of aberration. The direction of aberration refers to a direction of a component with a largest absolute value among all coefficients of all the blurred images when the aberrations added to the blurred images displayed simultaneously are expanded into Zernike polynomials.

In the blurred image preparation step S101, when selecting the added aberration to the original image, for example, it may be arbitrarily selected from among the aberrations caused by a standard progressive power lens.

### (Blur sensitivity measurement step S102)

The blur sensitivity measurement step S102 is a step of measuring the subject's sensitivity to aberration, for example, by simultaneously presenting multiple blurred images to the subject to compare the appearances of them and obtaining a subjective response of the subject. Specifically, for example, the blurred image 10A and the blurred image 10B as illustrated in FIG. 2 are simultaneously presented to a subject to compare the appearances of them and select which one appears clearer. Then, in the case of selecting the blurred image 10A in which the amount of added aberration is small (hereinafter, also referred to as a correct selection), it may be determined that the sensitivity to blur is high, and in the case of selecting the blurred image 10B in which the amount of added aberration is large (hereinafter, also referred to as an incorrect selection), it may be determined that the sensitivity to blur is low. In this specification, "simultaneously presenting multiple blurred images" means presenting multiple blurred images so that the multiple blurred images are present within the field of view of the subject and are visible to the subject, and the timing of starting or ending the presentation of each blurred image is not limited (for example, the timing of starting or ending the presentation of the blurred image 10A and the timing of starting or ending the presentation of the blurred image 10B may be different from each other).

However, when the sensitivity to blur is judged based on a single subjective response, for example, this may include the case where a blurred image in which the amount of aberration is small, happens to be selected even when a difference in appearance is not actually known. Therefore, it is preferable that in the blurred image preparation step S101 of this embodiment, multiple similar blurred images similar to the multiple blurred images are further prepared, and in the blur sensitivity measurement step S102, the multiple similar blurred images in which the amount of added aberration (preferably the amount only) is varied, are simultaneously presented to the subject, then, the subject is allowed to compare the appearances of the images, and the subjective response of the subject is obtained multiple times, to thereby measure the consistency of the subjective response of the subject. The similar blurred image will be described in detail below.

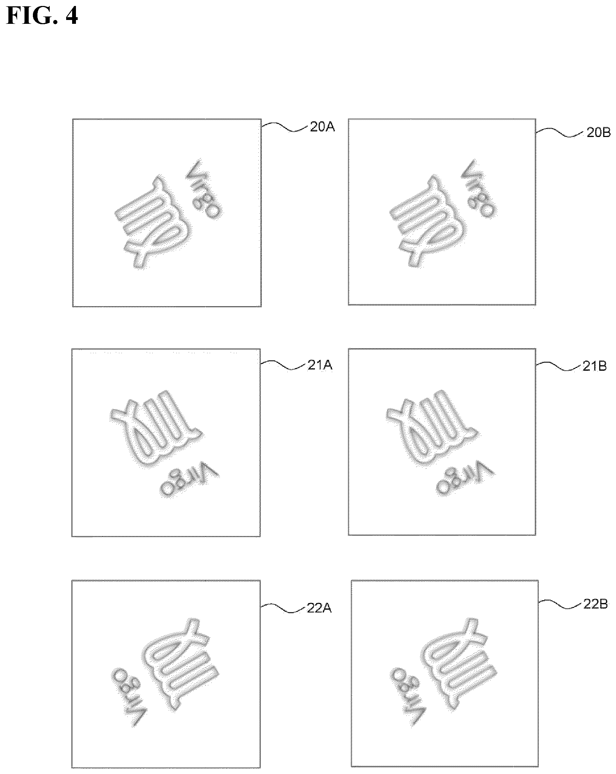

FIG. 4 illustrates an example of the similar blurred images similar to the blurred image 10A and the blurred image 10B. In this specification, the similar blurred image means, for example, an image obtained by rotating the blurred image at an arbitrary angle, or an image obtained by enlarging or reducing the blurred image at an arbitrary magnification. FIG. 4 illustrates similar blurred images 20A and 20B which are obtained by rotating the blurred images 10A and 10B by 90 degrees respectively, similar blurred images 21A and 21B which are obtained by rotating the blurred images 10A and 10B by 180 degrees respectively, and similar blurred images 22A and 22B which are obtained by rotating the blurred images 10A and 10B by 270 degrees respectively. In this case, for example, in the blur sensitivity measurement step S102, two (similar) blurred images having the same rotation angle (e.g., similar blurred image 20A and similar blurred image 20B) are simultaneously presented to the subject to compare the appearances of them, and the subject is given selections four times as to which one is more clearly visible, and subjective responses of the subject can be obtained four times. Then, for example, in the case of selecting the images in which the amount of added aberrations are small each time (blurred image 10A, similar blurred images 20A, 21A, 22B), it is determined that the consistency of the subjective response is high, and in the case of selecting the image in which the amount of added aberration is small and the image in which the amount of added aberration is large in roughly equal proportions, it is determined that the consistency of the subjective response is low. The high consistency of the subjective response confirms the high sensitivity to added aberration, and measuring the consistency of the subjective response allows a more accurate measurement of the sensitivity to blur. This makes it easier to determine a spectacle lens suitable for the subject.

It is important to note that although the blurred image and the similar blurred image have a common original image, they are not exactly the same images. When subjective responses are obtained multiple times using exact the same blurred images, the influence of the subject's adaptability will be strong, and it may be impossible to accurately measure the sensitivity to blur. In contrast, by using similar blurred images, the influence of the subject's adaptability can be reduced and the sensitivity to blur can be measured more accurately. Further, in the blur sensitivity measurement step S102, in order to further reduce the influence of the adaptability of the subject, the display positions of the image in which the amount of added aberration is small and the image in which the amount of added aberration is large may be switched and presented.

To measure the subject's sensitivity to blur in more detail, it is preferable that in the blurred image preparation step S101 of this embodiment, multiple difficulty level-varied blurred images are prepared, in which the difference in appearance is easier or harder to distinguish than the multiple blurred images; and in the blur sensitivity measurement step S102, multiple difficulty level-varied blurred images are simultaneously presented to the subject to compare the appearances of them, and the subjective response of the subject is obtained multiple times, to thereby measure the subject's sensitivity to aberration. The difficulty level-varied blurred images will be described in detail below.

When multiple blurred images in which the amount of the added aberration is varied, are simultaneously presented with respect to a predetermined original image, there are blurred images that clearly show the difference in appearance and blurred images that are difficult to tell the difference in appearance. In this specification, the ease (or difficulty) of understanding the difference in appearance of such blurred images is expressed as a difficulty level. The difficulty level of the blurred image depends on the amount and direction of the added aberration, as well as the spatial frequency characteristics of the original image. Therefore, for example, simply because there is a large difference in the amount of aberration added to two blurred images does not necessarily mean that the difficulty level is low. To determine the difficulty level of the blurred image, for example, VSOTF may be calculated for each of the multiple blurred images, and the difficulty level may be determined based on the difference between the VSOTFs, or the difficulty level may be determined based on the percentage of correct answers given by allowing a large number of subjects to compare the appearances of the images. The VSOTF is described in the following document: Thibos LN, Hong X, Bradley A, Applegate RA. Accuracy and precision of objective refraction from wavefront aberrations. J Vis. 2004 Apr 23;4(4):329-51. Description thereof will be omitted here.

By investigating to what difficulty level the subjects could distinguish the difference in appearance using multiple types of difficulty level-varied blurred images, the subject's sensitivity to blur can be measured in stages. This makes it easier to determine a spectacle lens suitable for the subject. In order to reduce the influence of the subject's adaptability, it is preferable that the multiple types of difficulty level-varied blurred images each has a different original image. Further, from the viewpoint of performing the measurement smoothly, it is preferable that the blurred image presented first (difficulty level-varied blurred images) in the blur sensitivity measurement step S102 is the blurred image with a low difficulty level. Further, as for the multiple types of difficulty level-varied blurred images, it is preferable to prepare similar blurred images for each type and obtain the subjective response of the subject multiple times, as described above, to thereby measure the consistency of the subjective response.

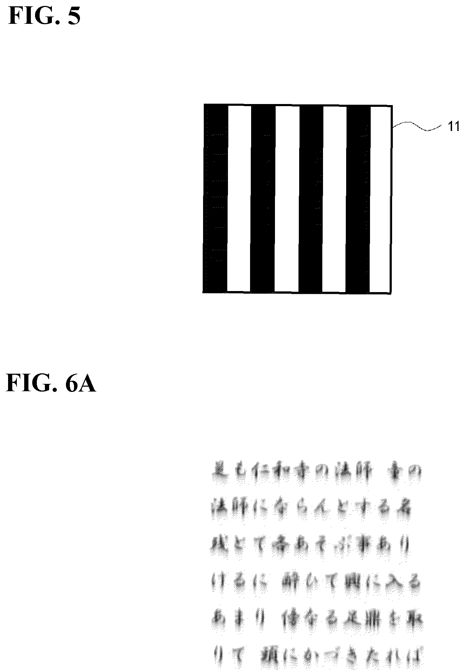

FIG. 5 is a view for explaining characteristic directions of the spatial frequency characteristics of an original image. In the original image 11 illustrated in FIG. 5, since the contrast changes significantly in the left-right direction on the paper, it can be said that the characteristic direction of the spatial frequency characteristics is the left-right direction. For example, when vertical aberration is added to such the original image 11, it is expected that the appearance of the resulting blurred image is not much different from the original image 11. That is, when the direction of the added aberration is different from the characteristic direction of the spatial frequency characteristics of the original image, this tends to result in a blurred image with high difficulty level. On the other hand, for example, when aberration in the left-right direction is added to the original image 11, this may result in a blurred image that looks significantly different from the original image 11. That is, when the direction of the added aberration coincides with the characteristic direction of the spatial frequency characteristics of the original image, this results in a blurred image with low difficulty level. Accordingly, in the blurred image preparation step S101 of this embodiment, it is preferable to select the original image according to the characteristic direction (direction of aberration) of the decrease in the spatial frequency characteristics due to the added aberration. This allows the difficulty level of the blurred image to be appropriately controlled. Specifically, for example, by selecting the original image so that the direction of the added aberration approximately coincides with the characteristic direction of the spatial frequency characteristics of the original image, the blurred image with extremely high difficulty level that is so blurry that no one can tell the difference in appearance, can be avoided. In this specification, the direction of the added aberration substantially coincides with the characteristic direction of the spatial frequency characteristics of the original image, which may mean, for example, that the absolute value of the inner product of the unit direction vectors of both directions is 0.7 or more. Further, the direction of aberration (0° to 180°) is the direction in which blurring increases, which is obtained from the direction in which the dispersion of the point spread function is greatest, for example. When using an original image that does not have a linear symmetry, a further code may be added based on the direction from the peak point of the point spread function to the center of gravity (that is, the direction of the aberration is -180° to 180°).

In the blur sensitivity measurement step S102, for example, it is preferable to present multiple blurred images with a size such that the spatial frequency of the main parts of the multiple blurred images is 3 CPD or more and 9 CPD or less (equivalent to visual acuity of 0.1 to 0.3). For example, when a nearsighted subject is presented with a blurred image that is too small to be seen without wearing spectacles, due to the influence of factors such as spectacle accommodation error, it may not be possible to accurately measure the sensitivity to blur. In contrast, by presenting a blurred image that is large enough that most subjects can see it without wearing spectacles, individual differences in perception are significantly reflected, and therefore the subject's sensitivity to blur can be measured more accurately. In this specification, the main part of a blurred image means a characteristic part in the blurred image where the difference in appearance is easily distinguishable. Further, it is preferable to present multiple blurred images of the same size, but for example, even when there is a slight difference in the magnification percentage of the multiple blurred images (for example, the magnification rate of less than 5%), it is acceptable as long as the subject can distinguish the difference in appearance. Further, the size at which the spatial frequency of the main part of the blurred image is 3 CPD to 9 CPD (equivalent to visual acuity of 0.1 to 0.3) means, for example, that in the case of an original image composed of line drawings and text, the width of the main lines that forms the drawings and text is between 1/6 degree and 1/18 degree of a visual angle.

In the blur sensitivity measurement step S102, for example, it is preferable that the subject's head is not fixed and that the multiple blurred images are presented at a distance of 0.3 m to 2 m from the subject's eyes. When measurements are performed with the subject's head fixed, tension occurs and the subject is put under a lot of strain. Further, since tension in the head muscles and eye muscles are linked, the subjects found it difficult to use their eyes in everyday situations, and the sensitivity to blur may not be measured accurately. In contrast, by performing measurements without fixing the subject's head, the strain on the subject can be reduced. Further, the sensitivity to blur can be measured more accurately by presenting multiple blurred images at a distance where the effect of slight changes in head position (for example, about 3 cm) is negligible.

The answer options for the subjective response in the blur sensitivity measurement step S102 may include an option such as "I can't tell the difference" in addition to "which image is clearly visible". The subject who answered "I can't tell the difference" at a frequent rate tends to be aware of having low sensitivity to blur. Further, when there are a plurality of subjects who selected the correct answer at roughly the same rate, the subject who answered "I can't tell the difference" at a frequent rate tends to be more tolerant of spectacle aberration than the subject who answered "I can't tell the difference" at not a frequent rate. In this way, by taking into consideration the response rate of "I can't tell the difference," it becomes easier to determine a spectacle lens that is more suitable for the subject in the spectacle lens determination step S103.

The sensitivity to blur (or aberration) can be divided into, for example, sensitivity to the size of the blur and the strength of a bias caused by the direction of the blur. The sensitivity to blur described above is mainly the sensitivity to the size of the blur, but in the blur sensitivity measurement step S102, it is preferable to measure not only the sensitivity to the size of the blur but also the strength of the bias caused by the direction of blur. Hereinafter, the direction of blur refers to a direction in a range of -180° to 180°, which corresponds to the direction of the spot image in the appearance of trailing illustrated in FIG. 3. That is, for example, the left-right direction is distinguished into the right direction and the left direction.

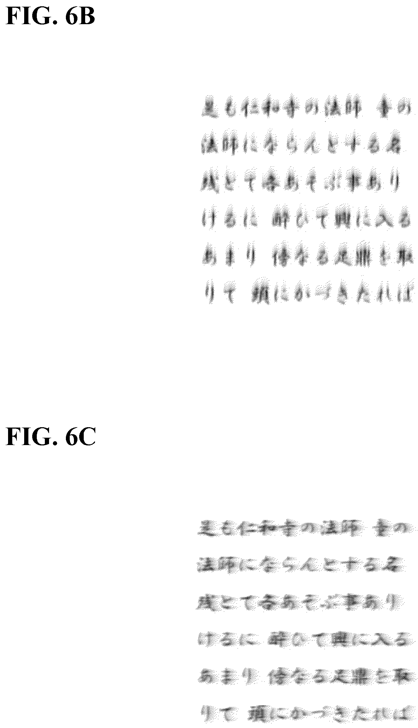

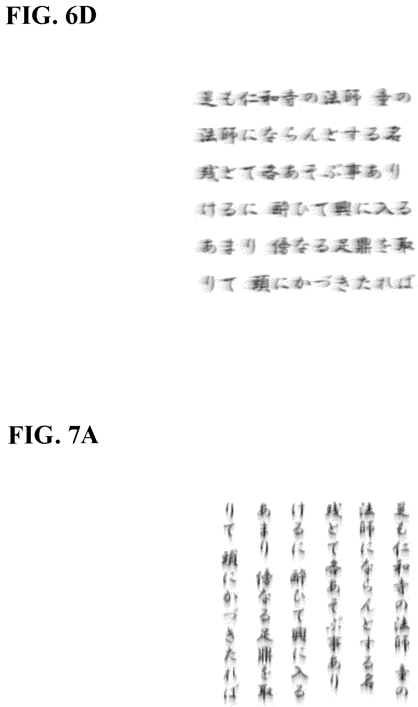

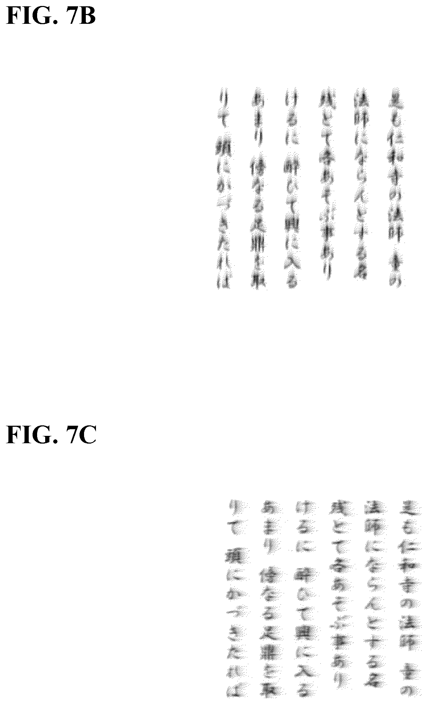

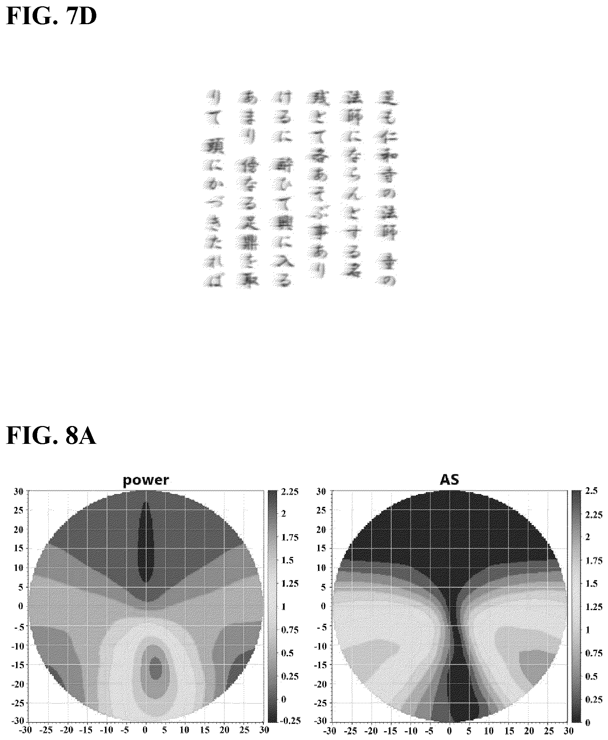

FIG. 6A to 6D and 7A to 7D are views illustrating examples of the blurred images for explaining the bias caused by the direction of blur. FIG. 6A is an image with aberration added downward, FIG. 6B is an image with aberration added upward, FIG. 6C is an image with aberration added rightward, and FIG. 6D is an image with aberration added leftward, with respect to an image (original image) of the text to be read rightward on the paper. Further, FIG. 7A is an image with aberration added downward, FIG. 7B is an image with aberration added upward, FIG. 7C is an image with aberration added rightward, and FIG. 7D is an image with aberration added leftward, with respect to an image (original image) of the text to be read downward on the page.

Among FIGS. 6A to 6D, many subjects selected FIG. 6C in which rightward aberration is added, as being clearly visible, and among FIGS. 7A to 7D, many subjects selected FIG. 7A in which downward aberration is added, as being clearly visible. That is, in the blurred image containing text, the blur tends to be less noticeable when the direction of reading the text matches the direction of the added aberration. Since the strength of such a bias varies from person to person, it is preferable to measure to what extent the subject has a bias caused by the direction of blur. It is also preferable to perform measurement while taking into consideration the difference due to the language that the subject normally uses.

As described above, the sensitivity to the size of blur can be measured, for example, by presenting multiple blurred images in which different amounts of aberrations are added, to a subject and judging whether the subject can distinguish the difference. Here, from the viewpoint of reducing the influence of the bias caused by the direction of blur and more accurately measuring the sensitivity to the size of the blur, for example, it is preferable to present the subject with blurred images in which the direction of the added aberration is varied by 180°, and to obtain a subjective response multiple times. Further, the extent of the bias caused by the direction of blur can be estimated, for example, from the extent of matching and mismatching in the answers when the direction of the added aberration is varied by 180°.

Also, regarding the inner product of the unit direction vector in the direction of the added aberration and in the characteristic direction of the spatial frequency characteristics of the original image, it is preferable to present the subject with a blurred image with the inner product of 0.7 or more and a blurred image with the inner product of -0.7 or less and obtain a subjective response multiple times, from the viewpoint of reducing the influence of the bias caused by the direction of blur and more accurately measuring the sensitivity to the size of the blur. Specifically, for example, for the original image of the text to be read rightward, it is preferable to prepare approximately equal numbers of blurred images with rightward aberration and blurred images with leftward aberration and present them to the subject.

### (Spectacle lens determination step S103)

The spectacle lens determination step S103 is, for example, a step of determining a spectacle lens suitable for the subject based on the subject's sensitivity to aberration measured in the blur sensitivity measurement step S102. In the spectacle lens determination step S103, it is preferable to determine a progressive power lens. This is because the influence of aberration is large in the progressive power lens, and therefore it is particularly important to take into consideration the subject's sensitivity to blur.

Specifically, for example, in the blur sensitivity measurement step S102, for the subjects who select correct answers many times, it may be determined that they have a high sensitivity to blur, and a progressive power lens that retains as little aberration on a principal meridian as possible may be selected. Further for example, for the subjects who select incorrect answers many times, it may be determined that they have a high sensitivity to blur but prefer a state of large aberration, and a progressive power lens having the aberration preferred by the subject may be selected. Further for example, for the subjects whose selections are sometimes correct and sometimes incorrect and who answered that they could not tell the difference at a low frequent rate, it may be determined that the subject has a low sensitivity to blur but does not consciously recognize this as being low, and a progressive power lens of a balanced design in which other improvement is achieved instead of a low aberration (for example, a design that prioritizes dioptric power) may be selected. Further for example, for the subjects who answer that they cannot tell the difference at a high frequent rate, it may be determined that they have a low sensitivity to blur, and a progressive power lens of a balanced design in which other improvement is achieved instead of retaining aberration on the principal meridian, may be selected.

In the vicinity of the principal meridian of a progressive power lens having a positive addition power, downward aberration occurs. Accordingly, in the spectacle lens determination step S103, the progressive power lens may be determined as follows, taking into consideration the bias caused by the direction of blur. For example, for the subjects who have high sensitivity to the size of blur and have a small bias caused by the direction of blur, it may be determined that the subjects are susceptible to blur (or have high sensitivity to blur) (or have high sensitivity to blur) on the principal meridian, and a progressive power lens of a design in which as little aberration as possible is retained on the principal meridian may selected. Further for example, for the subjects who have high sensitivity to the size of the blur and have a large bias caused by the direction of blur, it may be determined that blur on the principal meridian is hardly noticeable (but peripheral blur is noticeable), and a progressive power lens of a design in which the peripheral aberration is improved instead of retaining aberration on the principal meridian, may be selected. Further for example, for the subjects who have low sensitivity to the size of the blur and have small bias caused by the direction of blur, it may be determined that blur on the principal meridian is less noticeable (or the sensitivity to blur is low), and a progressive power lens of a design in which the peripheral aberration is improved instead of retaining aberration on the principal meridian, may be selected. Further for example, for the subjects who have low sensitivity to the size of the blur and a large bias caused by the direction of blur, it may be determined that the blur is hardly noticeable, and a progressive power lens of a design in which other aspect (depth of focus, etc.) is improved instead of retaining aberration on the principal meridian, may be selected.

In the spectacle lens determination step S103, the spectacle lens may be determined taking into consideration the subject's residual refractive error. Specifically, for example, for the subjects having severe refractive errors such as astigmatism, the spectacle lens may be selected on the assumption that they actually have a slightly higher sensitivity than the sensitivity measured in the blur sensitivity measurement step S102, taking into consideration the possibility that the difference in the appearance of the blurred images may be difficult to distinguish.

### (2) Spectacle lens determination support system

The present invention can also be applied as a determination support system for a spectacle lens. The spectacle lens determination support system of this embodiment includes, for example: a storage unit for storing multiple blurred images in which the amount of the added aberration is varied with respect to a predetermined original image; a display unit that simultaneously presents the multiple blurred images to a subject to compare appearances of the images; an input unit into which a subjective response of the subject is inputted; a judgement unit that judges the subject's sensitivity to aberration from the subjective response of the subject; and an output unit from which information for determining a spectacle lens suitable for the subject is outputted based on the subject's sensitivity to aberration. The spectacle lens determination support system of this embodiment can be realized, for example, by a tablet terminal equipped with a predetermined program, and has an advantage of low implementation costs. Further, the spectacle lens determination support system of this embodiment may further include, for example, a creation unit that adds a predetermined aberration to the original image and creates a blurred image.

### <Other embodiments of the present invention>

Although the embodiment of the present invention has been specifically described above, the present invention is not limited to the above-described embodiment, and various modifications can be made without departing from the gist of the present invention.

For example, the above-described embodiment shows a case where two blurred images are prepared, in which the amount of added aberration is varied with respect to a predetermined original image, and the two blurred images are simultaneously presented to the subject to compare the appearances of them. However, three or more blurred images may be prepared and presented simultaneously to compare the appearances of them. However, when three or more blurred images are presented simultaneously to compare the appearances of them, there is a possibility that difference due to a position within a visual field will become dominant. This may make it difficult for the subject to make judgment. Therefore, from the viewpoint of reducing the difference due to the position in the visual field and presenting the blurred images that are easy for the subject to judge, it is preferable to present two blurred images simultaneously to compare the appearances of them as in the above-described embodiment.

Further for example, the above-described embodiment shows a case where the directions of the added aberrations in the multiple blurred images presented simultaneously are substantially the same. However, the directions of the added aberrations in the multiple blurred images presented simultaneously do not necessarily have to be substantially the same. Specifically, for example, no astigmatism may be added to one blurred image (with no directional aberration) and astigmatism may be added to the other blurred image (with a directional aberration). In this case also, as in the above-described embodiment, the subject can easily compare the appearances of the multiple blurred images.

### Example

Next, examples of the present invention will be described. These examples are merely examples of the present invention, and the present invention is not limited to these examples.

### (Blurred image preparation step S101)

In this example, two blurred images were presented simultaneously. First, six different original images were prepared, and six types of blurred image pairs with different difficulty levels (difficulty level-varied blurred images) were prepared. Further, for each of the six types of blurred image pairs, a rotation angle was changed, and three types of similar blurred images (four types including the original blurred image) were prepared, and a total of 24 types of blurred image pairs were prepared.

### (Blur sensitivity measurement step S102)

In this example, blurred image pairs were displayed on the tablet terminal 24 times in random order, and the subject was allowed to compare the appearances of them, and subjective response as to which image appeared clearer were obtained 24 times. The blurred image was enlarged to a size such that the spatial frequency of the main parts was about 6 CPD (equivalent to visual acuity of 0.2), and was presented at a distance of 1 m in front of the subject.

In this example, the subjects who selected blurred images in which the amount of added optical aberration is small (or blurred images having good evaluation indexes) at a frequent rate (for example, 18 or more times out of 24 times) were classified as group A having high sensitivity to aberration, and the subjects who selected blurred images in which the amount of added optical aberration is small (or blurred images with good evaluation indexes) at a moderate frequent rate (for example, 7 to 17 times out of 24 times) were classified as group B having low sensitivity to aberration, and the subjects who selected blurred images in which the amount of added optical aberration is small (or blurred images with a good evaluation index) at a not frequent rate (for example, 6 times or less out of 24 times) were classified as group C who evaluated the images from a perspective different from a designer. In this specification, the evaluation index is an index indicating the visibility of a blurred image, calculated from the VSOTF of the blurred image or from the past selection results of the subjects.

### (Spectacle lens determination step S103)

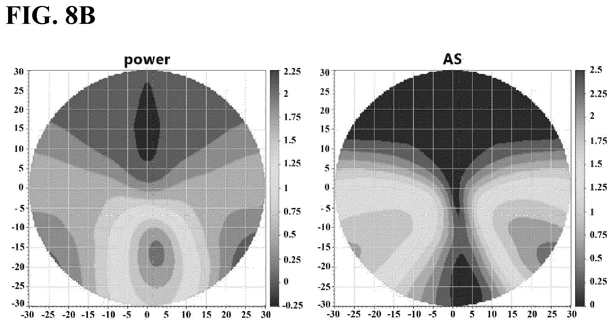

In this example, a progressive power lens suitable for the subject was determined based on the subject's sensitivity to aberration measured in the blur sensitivity measurement step S102 described above. Specifically, for example, for the group A, a progressive power lens having a power distribution (power) and an astigmatism distribution (AS) as illustrated in FIG. 8A was determined, and for the group B, a progressive power lens having a power distribution (power) and an astigmatism distribution (AS) as illustrated in FIG. 8B was determined. This is because as illustrated in FIG. 8A, a lens that is free of astigmatism on and near the principal meridian is suitable for the subject having high sensitivity to aberration, and as illustrated in FIG. 8B, a lens with astigmatism on the principal meridian but with a wide range of near addition power on both sides are suitable for the subject having low sensitivity to aberration. In the measurement performed by the present inventor so far, no subjects that fell into the group C were found, but since it is difficult to apply objective optical indexes to the group C, it is advisable to perform additional investigations, such as counseling, before determining a spectacle lens.

As described above, it was confirmed that the spectacle lens suitable for a subject can be determined by taking into consideration the subject's sensitivity to aberration.

### Description of signs and numerals

- 10, 11: Original image
- 10A, 10B: Blurred image
- 20A, 20B, 21A, 21B, 22A, 22B: Similar blurred images
- S101: Blurred image preparation step
- S102: Blur sensitivity measurement step
- S103: Spectacle lens selection step

## Claims

1. A method for determining a spectacle lens, the method comprising:
preparing multiple blurred images in which an amount of added aberration is varied with respect to a predetermined original image;
simultaneously presenting the multiple blurred images to a subject to compare appearances of them, then obtaining a subjective response of the subject, to thereby measure a subject's sensitivity to aberration; and
determining a spectacle lens suitable for the subject based on the subject's sensitivity to aberration.

2. The method for determining a spectacle lens according to claim 1,
wherein in the preparation of the multiple blurred images, multiple similar blurred images similar to the multiple blurred images are further prepared; and
in the measurement of the subject's sensitivity to aberration, the subject is simultaneously presented with the multiple similar blurred images to compare the appearances of them, and a subjective response of the subject is obtained multiple times to thereby measure a consistency of the subjective response.

3. The method for determining a spectacle lens according to claim 1,
wherein in the preparation of the multiple blurred images, difficulty level-varied multiple blurred images are further prepared so that a difference in appearance is easier or harder to distinguish than the above multiple blurred images; and
in the measurement of the subject's sensitivity to aberration, the subject is simultaneously presented with the difficulty level-varied multiple blurred images to compare appearances of them, and a subjective response of the subject is obtained multiple times, to thereby measure the subject's sensitivity to aberration.

4. The method for determining a spectacle lens according to claim 1,
wherein in the preparation of the multiple blurred images, the original image is selected according to a characteristic direction of a decrease in spatial frequency characteristics due to the added aberration.

5. The method for determining a spectacle lens according to claim 1,
wherein in the determination of the spectacle lens, a progressive power lens is determined.

6. The method for determining a spectacle lens according to claim 1,
wherein in the measurement of the subject's sensitivity to aberration, the multiple blurred images are presented with a size such that a spatial frequency of main parts of the multiple blurred images is 3 CPD or more and 9 CPD or less.

7. The method for determining a spectacle lens according to claim 1,
wherein in the measurement of the subject's sensitivity to aberration, a subject's head is not fixed, and the multiple blurred images are presented at a distance of 0.3 m or more and 2 m or less from subject's eyes.

8. A system for supporting determination of a spectacle lens, the system comprising:
a storage unit that stores multiple blurred images in which amounts of added aberrations are varied, with respect to a predetermined original image;
a display unit that simultaneously presents the multiple blurred images to a subject to compare appearances of them;
an input unit into which a subjective response of the subject is inputted;
a determination unit that determines a subject's sensitivity to aberration from the subjective response; and
an output unit from which information for determining a spectacle lens suitable for the subject is outputted based on the sensitivity to aberration.
